# EUROPEAN PATENT SPECIFICATION

(11) **EP 1 383 497 B1**
(45) Date of publication and mention of the grant of the patent: **15.10.2008**
(21) Application number: 02725885.4
(22) Date of filing: 02.05.2002
(51) Int. Cl.: A61K 31/4164, A61K 31/4178, A61P 25/28

(54) **CARBONIC ANHYDRASE ACTIVATORS FOR ENHANCING LEARNING AND MEMORY**
KOHLENSÄUREANHYDRASE-AKTIVATOREN ZUR VERBESSERUNG DER LERN- UND GEDÄCHTNISLEISTUNG
ACTIVATEURS D'ANHYDRASE CARBONIQUE AMELIORANT L'ACQUISITION DES CONNAISSANCES ET LA MEMORISATION

(30) Priority: 02.05.2001 US 287721 P
(43) Date of publication of application: 28.01.2004
(60) Divisional application: 08003718.7
(73) Proprietor: Blanchette Rockefeller Neurosciences Institute, Rockville, MD 20850 (US)
(72) Inventor: ALKON, Daniel, Bethesda, MD 20817 (US); SUN, Miao-Kun, Bethesda, MD 20878 (US)
(74) Representative: Kador & Partner
(86) International application number: PCT/US2002/013784
(87) International publication number: WO 2002/087423

(56) References cited:
- EP-A1- 0 387 867
- WO-A-99/52519
- WO-A1-00/66617
- WO-A1-85/03869
- WO-A2-00/40241
- WO-A2-01/93744
- RO-A- 86 393
- US-A- 5 075 338
- US-A- 5 220 068
- US-A- 5 972 684
- US-A- 6 043 283
- MIAO-KUN SUN ET ALL.: "Pharmacological enhancement of synaptic efficacy, spatial learning, and memory through carbonic anhydrase activation in rats" THE JOURNAL OF PHARMACOLOGY AND EXPERIMENTAL THERAPEUTICS, vol. 297, no. 3, June 2001 (2001-06), pages 961-967, XP002349546
- SUN ET AL.: 'Pharmacological enhancement of synaptic efficacy, spacial learning and memory through carbonic anhydrase activation in rats' PHARMACOLOGY AND EXPERIMENTAL THERAPEUTICS vol. 297, no. 3, June 2001, pages 961 - 967, XP002959205
- SUN ET AL.: 'Theta rhythm of hippocampal CA1 neuron activity: gating by GABAergic synaptic depolarization' THE JOURNAL OF NEUROPHYSIOLOGY vol. 85, no. 1, January 2001, pages 269 - 279, XP002959204
- SUPURAN ET AL.: 'Carbonic anhydrase activators: synthesis of high affinity isozymes I, II and IV activators, derivatives of 4-(arylsulfonylureido-amino acid)ethyl-1H-imidazole' ENZYME INHIBITION vol. 15, no. 5, 2000, pages 471 - 486, XP002959217
- BRIGANTI ET AL.: 'Carbonic anhydrase activators: X-ray crystallographic and spectroscopic investigations for the interaction of isozymes I and II with histamine' BIOCHEMISTRY vol. 36, 1997, pages 10384 - 10392, XP002959203
- YASAR S. ET AL: 'Are metabolites of l-deprenyl (selegiline) useful or harmful? Indications from preclinical research' J. NEURAL TRANSM. vol. 48, 1996, pages 61 - 73
- ZHONG CHEN ET AL: 'Effects of histamine on MK-801-induced memory deficits in radial maze performance in rats' BRAIN RESEARCH vol. 839, 1999, pages 186 - 189
- DATABASE WPI Week 200002, Derwent Publications Ltd., London, GB; AN 2000-021666 & RU 2 115 653 A (AS SIBE IRKUT ORGANIC CHEM INST) 20 July 1998

## Description

### BACKGROUND OF THE INVENTION

This invention relates to the use of compositions for manufacture of a drug to improve attention, learning, and memory by activating carbonic anhydrase. Drugs that enhance acquisition and/or recall of associative memory represent important goals in the therapy of cognitive disorders. The effectiveness of such therapy depends on whether the targeted mechanisms are actually involved in memory itself. Learning and memory are believed to require modifications of synaptic strength among relevant neurons in the network, through an interaction of multiple afferent pathways and signal molecules (Christie et aL, 1994; Komhauser and Greenberg, 1997; Ohno et al., 1997; Alkon et aL, 1998; Paulsen and Moser, 1998; Xiang et al., 1998 Tang et al., 1999; Wu et al., 2000). A requirement for multiple synaptic interactions, versus a single glutamatergic pathway often studied experimentally, is in fact consistent with characterization of multiple deficits of neurotransmitters in memory impairments, including Alzheimer's disease. Targeting the relevant synaptic/sigal interactions within memory traces therefore might be an effective way to achieve a specific effect on learning and memory pharmacologically.

In mammals, the essential role of hippocampal CA1 pyramidal cells in spatial memory is well established. The CA1 pyramidal cells receive, in addition to glutamatergic input from the CA3 pyramidal neurons, abundant cholinergic and GABAergic inputs. Activation of the medical septal afferents within the perforant pathway, a major cholinergic input to the hippocampus (Cooper and Sofroniew, 1996), is believed to be required for associative learning (Dickinson-Anson et al., 1998; Perry et al., 1999), since its disruption abolishes spatial memory (Winson, 1978; Winkler et al., 1995). GABAergic interneurons, on the other hand, control hippocampal network activity and synchronize the firing of pyramidal cells (Buhl et al., 1995; Cobb et al., 1995; Banks et aL, 2000). One GABAergic interneuron is known to innervate some 1000 pyramidal cells, effectively shutting down the signal outflow when the interneurons are active (Sun et al., 2000). The functional interaction between these major inputs thus plays a significant role in hippocampus-dependent memory (Bartus et aL, 1982; Winkler et al., 1995; Paulsen and Moser, 1998) and has attracted much attention in an effort to "dissect" the memory traces.

Consistent with the observations that the GABAergic synaptic responses can be switched from inhibitory to excitatory (Alkon et al., 1992; Collin et al., 1995; Kaila et al., 1997; Taira et al., 1997; Sun et al., 2000, 2001b), evidence has been provided that such a synaptic switch depends on the increased HCO₃ conductance through the GABA_{A} receptor-channel complex and dramatically alters the operation of signal transfer through the hippocampal network (Sun et al., 1999, 2000). The synaptic switch appears to depend on carbonic anhydrase, a zinc-containing enzyme that catalyzes the reversible hydration of carbon dioxide. Carbonic anhydrase is present within the intracellular compartments of the pyramidal cells (Pasternack et al., 1993). The fact that a membrane-impermeant carbonic anhydrase inhibitor, benzol amide, was effective in blocking the synaptic switch when introduced into the recorded pyramidal cells, but not when applied extracellularly (Sun et al., 1999), indicates that the underlying enzyme is intracellular. Blocking the rapid HCO₃ formation that depends on carbonic anhydrase activity thus prevents the synaptic switch in vitro and impairs rat spatic plasticity and memory.

Acetazolamide, a known inhibitor of carbonic anhydrase activity, inhibits theta rhythm, learning, and memory. Sun MK, Zhao WQ, Nelson TJ, Alkon DL., "Theta Rhythm of Hippocampal CA1 Neuron Activity: Gating by GABAergic Synaptic Depolarization," J Neurophysiol 2001 Jan;85(1):269-79. Prior data showing that inhibition of carbonic anhydrase activity impaired memory formation was not predictive that activation would enhance memory formation. For example, it was not known if the enzyme was already operating at a maximal level in neurons involved with learning, which could not be further activated. It was also not known if there are homeostatic mechanisms in such cells that would neutralize any activation due to administration of a compound according to the invention.

Pending patent application PCT/US01/18329, filed June 7, 2001 by the National Institutes of Health, disclosed that activating carbonic anhydrase can lead to improved learning and memory. There is a long-felt need for compounds and pharmaceutical agents that activate carbonic anhydrase and improve learning and memory in mammals.

Z. Chen et al., report in Brain Research 839 (1999) 186-189 on effects of histamine on MK-801-induced memory deficits in radial maze performance in rats.

WO 00/66617 A1 discloses methods of diagnosis and treating Alzheimer's disease or other neuronal atrophy-associated dementia using i.a. 5-methyladenine, leucine, mistidine or vinblastine as effective compound RO-A 86 393 and RU-A-2 115 653 disclose imidazole compounds in pharmaceutical compositions.

The invention provides the use of compounds for manufacture of drugs for improving attention and/or memory acquisition comprising stimulating intraneuronal carbonic anhydrase activity as specified in claim 1. The stimulation is achieved by administering a carbonic anhydrase activator. The use allows treating neurodegenerative disorders to enhance cognitive ability, treating dementia, and also enhancing attention and learning in healthy individuals.

The invention provides the use of compound for manufacture of a drug for treatment of attentive cognition wherein the compound potentiates intraneuronal carbonic anhydrase activity thereby improving establishment of a theta rhythm.

The invention relates to the use of a compound according to claim 1.

The carbonic anhydrase activator may be administered as a pharmaceutical composition or in a pharmaceutically acceptable carrier.

The patient may have a neurodegenerative disorder or the use enhances cognitive ability, attention, learning, and/or memory in individuals without a neurological disorder.

The use of the compound according to claim 1 facilitates establishment of a theta rhythm via bicarbonate-mediated GABAergic depolarization. The use of the compound according to claim 1 improves memory formation, learning, spatial memory, and/or attention. The use of the compound according to claim 1 intervenes in the intracellular signaling cascade responsible for theta rhythm, the intervention comprising modulating HCO₃⁻ conductance by directly altering intraneuronal carbonic anhydrase activity. The intervention may modulate the HCO₃⁻ current relative to the Cl⁻ and K⁺ currents.

The use of the compound may improve attentive cognition in a subject with Alzheimer's disease, stroke, hypoxia, and/or ischemia.

The use of the compound may employ a compound that provides carbonic anhydrase activity at least 150%, 200%, or 250% that of alanine in vitro.

Also disclosed is an article of manufacture comprising a pharmaceutical composition comprising an activator compound packaged together with labeling indicating use for improving attentive cognition, the activator compound being effective to enhance brain carbonic anhydrase activity and selected from structures as defined in claim 1 or salts thereof.

Further objectives and advantages will become apparent from a consideration of the description, drawings, and examples.

### BRIEF DESCRIPTION OF THE DRAWINGS

The invention is better understood by reading the following detailed description with reference to the accompanying figures:

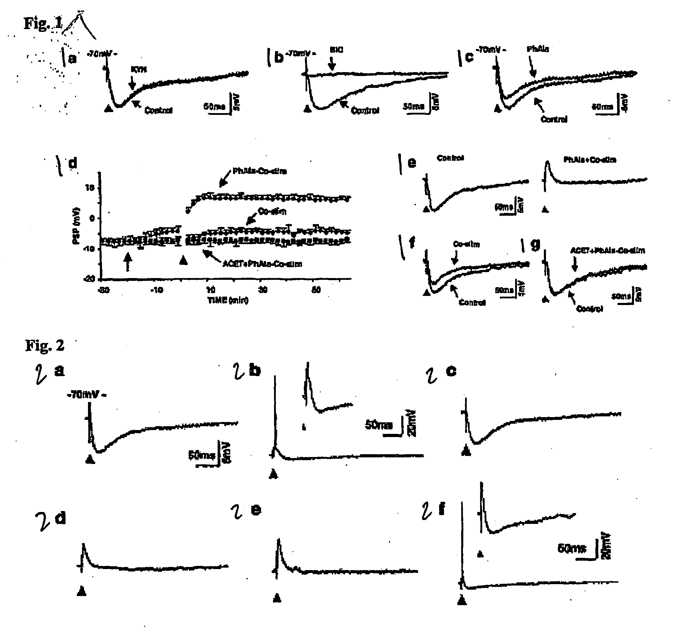

Figures 1a, 1b, 1c, 1d, 1e, 1f and 1g demonstrate the associated activation of cholinergic and GABAergic inputs and carbonic anhydrase induced long-term synaptic switching from an inhibitory to excitatory response. Single-pulse stimulation of stratum pyramidale (50 µA, 50 µs) evokes an IPSP (control), which is not changed by bath kynurenic acid (KYN; 500 µM, 20 min; Fig. 1a). The IPSP (control), however is eliminated by bicuculline (BIC; 1 µM, 30 min; Fig. 1b). The application of phenylalanine (reference) (100 µM, starting at the vertical arrow in d) reduces the IPSP slightly when applied alone (Fig. 1c) but induces a lasting synaptic reversal of the GABAergic responses when association with costimulation (at the arrowhead in Fig. 1d; under *Materials and Methods*) of stratum oriens and stratum pyramidale (PhAla + Co-stim; Fig. 1d and Fig. 1e). The same costimulation, however, does not trigger the synaptic switch (Co-stim; Fig. 1d and Fig. 1f) and the effects of phenylalaline-costimulation on the synaptic switch are eliminated (ACET + PhAla-Co-stim; Fig. 1d and Fig. 1g) by the application of acetazolamide (reference) (10 µM, also starting at the vertical arrow in Fig. 1d). Arrowheads indicate the time when single-pulse stimulation of stratum pyramidale is delivered. In d, the data points are illustrated as means ± standard errors of the means and for clarity, only every other minute is illustrated.
Figures 2a, 2b, 2c, 2d, 2e and 2f shows how synaptic switch converts excitatory input filter into amplifier. Single-pulse stimulation of stratum pyramidale evokes an IPSP (Fig. 2a). Single pulse stimulation of Sch at above-threshold intensity evokes an action potential (Fig. 2b). Co-single-pulse stimulation of stratum pyramidale and Sch (the same as Fig. 2a and Fig. 2b) eliminates the EPSP and no action potential is evoked (Fig. 2c). After the associated costimulation of stratum pyramidale and stratum oriens (under *Materials and Methods*) in the presence of phenlyalanine (reference), the IPSP is reversed to EPSP, observed at the same resting membrane potential (Fig. 2d). Single-pulse stimulation of Sch at below-threshold intensities evokes an EPSP (Fig. 2e). Cosingle-pulse stimulation of stratum pyramidale and Sch (the same as Fig. 2d and Fig. 2e) evokes an action potential (Fig. 2f). Arrowheads indicate the time when single-pulse stimulation of stratum pyramidale or costimulation is delivered. The calibration bar units are the same for the traces and insets (as in Fig. 2a) except Fig. 2b and Fig. 2f.

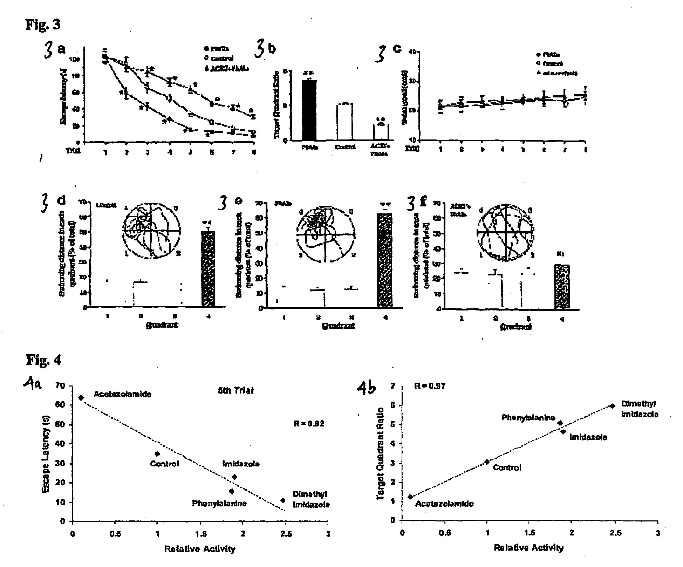

Figures 3a, 3b, 3c, 3d, 3e and 3f demonstrate how the carbonic anhydrase activator enhances rat performance in the hidden platform water maze task. The figure illustrates escape latency (means ± standard errors of the means; *n* = 10 for each group) in water maze training (Fig. 3a) across eight trials (*F*_{7,105} = 55.78, *p* < 0.0001), swim speeds (Fig. 3c), and quadrant preference (Fig. 3d-f) conducted at the end of the eighth training session. Quadrant 4 is the target quadrant during training. Insets are paths taken by representative rats with quadrant numbers indicated. The target ratio is defined as the time searching in the target quadrant/the average of the nontarget quadrants (Fig. 3b).
Figures 4a and 4b show a linear correlation between the relative activity of carbonic anhydrase in the presence of the activator compound and the escape latency (Fig. 4a), which reflects learning, and the target quadrant ratio (Fig. 4b) which reflects memory. Techniques were as described in the Examples. Phenylalanine and acetazolamide are reference compounds.

According to the invention, one can administer a drug to a patient at a given time to produce a cognitive effect (referred to as attentive cognition), such as learning, learning-related attention, associative learning, and memory acquisition, and memory consolidation (without effecting memory storage and recall) by activating neuronal carbonic anhydrase e.g. by compounds that enhance carbonic anhydrase activity and thereby switch GABAergic activity from predominantly hyperpolarizing Cl- conductance to a depolarizing, primarily HCO₃⁻ conductance, entraining pyramidal cells into a theta rhythm.

Principal aspects of the invention include (1) specific cognitive effects, (2) theta rhythm effects, and in particular, (3) the use of a compound according to claim 18 for enhancing learning by stimulating carbonic anhydrase activity above standard control levels. The fact that carbonic anhydrase is a common link between stimulating excitatory post synaptic potential and stimulating theta rhythm allows therapies for neurological disorders including cognitive therapy.

The invention provides the use of a compound according to claim 1 for improving attentive cognition comprising administering a compound that enhances intraneuronal carbonic anhydrase activity thereby effecting establishment of a theta rhythm. The metabolic pathway of the compound preferably involves bicarbonate-mediated GABAergic depolarization. The term "attentive cognition" is meant to encompass memory formation, learning, spatial memory, and attention. Attentive cognition can include one or more of attention, learning, and/or memory acquisition and/or retention. According to the invention, theta rhythm can be enhanced by carbonic anhydrase activators to treat neurological disorders such as stroke, hypoxia, and ischemia.

Administering a compound used in the invention to the train means administering the compound itself which crosses the blood brain barrier in an effective amount to deliver the compound to the brain.

Methods of measuring carbonic anhydrase activity and attentive cognition in rats have previously been published. Sun MK, Zhao WQ, Nelson TJ, Alkon DL., "Theta Rhythm of Hippocampal CA1 Neuron Activity: Gating by GABAergic Synaptic Depolarization," J Neurophysiol 2001 Jan;85(1):269-79, and

Sun MK, Alkon DL., "Pharmacological Enhancement of Synaptic Efficacy, Spatial Learning, and Memory Through Carbonic anhydrase Activation in Rats," J. Pharmacol. and Experimental Therapeutics 297(3):961-967. In the presence of carbonic anhydrase activators, co-microstimulation of cholinergic inputs from stratum oriens and gamma-aminobutyric acid (GABA)ergic inputs from stratum pyramidale at low intensities switched hyperpolarizing GABA-mediated inhibitory postsynaptic potentials to depolarizing responses. The carbonic anhydrase activators caused rats to exhibit superior learning of the Morris water maze task, suggesting that the GABAergic synaptic switch is critical for gating the synaptic plasticity that underlies spatial memory formation. Increased carbonic anhydrase activity enhances perception, processing and storing of temporally associated relevant signals and represents an important therapy for learning and memory pharmacology.

The carbonic anhydrase activators used according to the invention are selected from imidazole and their structural analogs or, derivatives as defined by formula I and salts thereof, as shown further by the exemplary embodiments described below. Tables 1 and 2 show exemplary compounds of the invention. The activities of these compounds relative to the control level of activity for the CA-II isozyme are also presented.

Suitable activator compounds and methods for measuring carbonic anhydrase activity can be found in Clare, B. W. and Supuran, C.T., "Carbonic anhydrase activators: 3: Structure-activity correlations for a series of isozyme II activators", J. Pharmaceut. Sci. 83: 768-773, 1994; Supuran, C.T., et al., "Carbonic anhydrase activators. Part 7. Isozyme II activation with bisazolylmethanes, -ethanes and related azoles.," Biol. Pharm. Bull. 16: 1236-1239, 1993; and Supuran, C.T., et al., "Carbonic anhydrase activators: XV. A kinetic study of the interaction of bovine isozyme II with pyrazoles, bis- and tris- azolyl-methanes.," Biol. Pharm. Bull. 19: 1417-1422,1996.

The activator compounds according to the invention are imidazole compounds and their structural analogs, derivatives and salts, having the general structure: 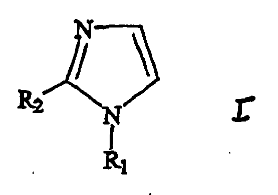 wherein R¹ and R² are independently H or linear, branched or cyclic C₁-C₆ alkyl or C₁-C₄ alkyl. Methyl and ethyl are examples of alkyl groups that may be in position R¹, or
wherein R¹ is 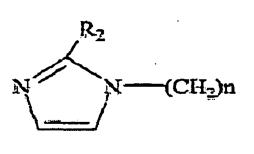 and*n* is 1 or 2 and R² is H or linear, branched or cyclic C₁-C₆ alkyl or C₁-C₄ alkyl. Methyl is an example of R².

Different R groups may heighten the activator effect and associated cognitive enhancement. Such enhanced effects are readily determined by routine experimentation. Examples of imidazole compounds of the invention (structure I, compounds 1-4) and linked di-imidazoles of the invention (compounds 5-25) are shown in Tables 1 and 2, respectively.

**Table 1**

| **Effector** | **R₁** | **R₂** | **%Activity** |
|---|---|---|---|
| 1 | H | H | 190 |
| 2 | CH₃ | H | 194 |
| 3 | C₂H₃ | H | 203 |
| 4 | CH₃ | C₂H₅ | 247 |

**Table 2**

| **Effector** | **n** | **R₂** | **%Activity** |
|---|---|---|---|
| 5 | 1 | H | 140 [di(1-imidazolyl)methane] |
| 6 | 1 | CH₃ | 169 [di(2-methyl-1-imidazolyl)methane] |
| 7 | 2 | H | 154 [1,2-di(1-imidazolyl)ethane] |
| 8 | 2 | CH₂ | 131 [1,2-di(2-methyl-1-imidazolyl)ethane] |

Some of these compounds were tested on rats in learning and memory experiments. The results are graphed in Figures 4a and 4b. The activation of carbonic anhydrase is shown to be directly related to learning and memory effects in a mammal. Reduced activity inhibits learning and memory. Increased activity improves learning and memory in a linear proportional manner.

Compounds of the invention are set forth in the above referenced Tables 1 and, 2 Many of these compounds are already known and the methods for obtaining them are known to persons of ordinary skill. The compounds may be combined and may be administered in a pharmaceutically acceptable carrier, and packaged together with labeling indicating a cognitive effect.

The compounds of the present invention may provide neuronal carbonic anhydrase activity of at least 110, 115, 125, 135, 150, 170, 180, 190, 200, 210, 220, 230, 240 and 250% that of alanine.

The effective dose for administration of the compounds is one that enhances carbonic anhydrase activity in cells of neuronal signaling pathways associated with learning particular tasks, attention, and memory. When the activator compounds are administered in effective doses according to the invention, they enhance carbonic anhydrase activity by either directly activating carbonic anhydrase or by inducing the calcium-signaling intracellular neuronal pathway to activate carbonic anhydrase. If a dose is too high, there is no beneficial learning effect and indeed the subject may demonstrate impaired learning. Thus, a large dose may overwhelm the neuronal pathways and a small dose may not achieve the desired enzyme activation and learning effect. The dosage must be adjusted to get the desired result.

Extrapolating from rat dosing, which is predictive of human dosing, effective doses of phenylalanine (reference) (50 mM) or imidazole (0.5 M) for treating humans may include the equivalent of 0.1, 0.3, 1, 3 or 10 ml/kg body weight taken twice per day. A desirable dosing regimen includes administering the compound about 30 minutes prior to desired attentive cognition activity.

The chemical compositions useful in the present invention can be "converted" into pharmaceutical compositions by the dissolution in, and/or the addition of, appropriate, pharmaceutically acceptable carriers or diluents. Thus, the compositions may be formulated into solid, semi-solid, liquid, or gaseous preparations, such as tablets, capsules, powders, granules, ointments, solutions, suppositories, injectables, inhalants, and aerosols, using conventional means. Known methods are used to prevent release or absorption of the active ingredient or agent until it reaches the target cells or organ or to ensure time-release of the agent. A pharmaceutically acceptable form is one which does not inactivate or denature the active agent. In pharmaceutical dosage forms useful herein, the present compositions may be used alone or in appropriate association or combination with other pharmaceutically active compounds.

Accordingly, the pharmaceutical compositions of the present invention can be administered to any of a number of sites of a subject and thereby delivered via any of a number of routes to achieve the desired effect. Local or systemic delivery is accomplished by administering the pharmaceutical composition via injection, infusion or sintillation into a body part or body cavity, or by ingestion, inhalation, or insufflation of an aerosol. Referred routes of administration include parenteral administration, which includes intramuscular, intracranial, intravenous, intraperitoneal, subcutaneous intradermal, or topical routes.

The present compositions can be provided in unit dosage form, wherein each dosage unit, *e*.*g*., a teaspoon, a tablet, a fixed volume of injectable solution, or a suppository, contains a predetermined amount of the composition, alone or in appropriate combination with other pharmaceutically active agents. The term "unit dosage form" refers to physically discrete units suitable for a human or animal subject, each unit containing, as stated above, a predetermined quantity of the present pharmaceutical composition or combination in an amount sufficient to produce the desired effect. Any pharmaceutically-acceptable diluent or carrier may be used in a dosage unit, *e*.*g*., a liquid carrier such as a saline solution, a buffer solution, or other physiologically acceptable aqueous solution), or a vehicle. The specifications for the novel unit dosage forms of the present invention depend on the particular effect to be achieved and the particular pharmacodynamic properties of the pharmaceutical composition in the particular host.

An "effective amount" of a composition is an amount that produces the desired effect in a host, which effect can be monitored, using any end-point known to those skilled in the art.

Furthermore, the amount of each active agent exemplified herein is intended to provide general guidance of the range of each component which may be utilized by the practitioner upon optimizing these methods for practice either *in vitro* or *in vivo*. Moreover, exemplified dose ranges do not preclude use of a higher or lower doses, as might be warranted in a particular application. For example, the actual dose and schedule may vary depending on (a) whether a composition is administered in combination with other pharmaceutical compositions, or (b) inter-individual differences in pharmacokinetics, drug disposition, and metabolism. Similarly, amounts may vary for in vitro applications. One skilled in the art can easily make any necessary adjustments in accordance with the necessities of the particular situation.

There are several isozymes of carbonic anhydrase. See Lindskog, "Structure and Function of Carbonic Anhydrase, "Pharmacol. Ther. Vol.74 (1), P1-20, 1997. The structure of the CAII binding site for acetazolamide and some other inhibitors is known. This knowledge allows rational design of derivatives and analogs of the compounds listed herein.

### EXAMPLE

### Introduction

CA1 pyramidal cells were recorded in rat hippocampal slices. In the presence of carbonic anhydrase activators, comicrostimulation of cholinergic inputs from stratum oriens and γ-aminobutyric acid (GABA)ergic inputs from stratum pyramidale at low intensities switched the hyperpolarizing GABA-mediated inhibitory postsynaptic potentials to depolarizing responses. In the absence of the activators, however, the same stimuli were insufficient to trigger the synaptic switch. This synaptic switch changed the function of the GABAergic synapses from excitation filter to amplifier and was prevented by carbonic anhydrase inhibitors, indicating a dependence on HCO₃. Intralateral ventricular administration of these same carbonic anhydrase activators caused the rats to exhibit superior learning of the Morris water maze task, suggesting that the GABAergic synaptic switch is critical for gating the synaptic plasticity that underlies spatial memory formation. Increased carbonic anhydrase activity also enhances perception, processing, and storing of temporally associated relevant signals and represents an important therapeutic target in learning and memory pharmacology.

### Materials and Methods

**Brain Slices.** Male Sprague-Dawley rats (150-180 g) were anesthetized with pentobarbital and decapitated. The hippocampal formation was removed and sliced (400 µm) with a McIllwain tissue chopper (Sun et al., 1999). Slices were maintained in an interface chamber (Medical systems Corp., Greenvale, NY) at 31°C with continuous perfusion of artificial cerebrospinal fluid. Artificial cerebrospinal fluid consisted of 125 mM NaCl, 3 mM KCI, 1.3 mM MgSO₄, 2.4 mM CaCl₂, 26 mM NaCHO₃, 1.25 mM NaH₂PO₄, and 10 mM C₆H₁₂O₆.

**Electrophysiology**. Intracellular recordings were obtained from CA1 pyramidal neurons using glass micropipette electrodes filled with 2 M potassium acetate (pH 7.25), with measured tip resistance in the range 70 to 120 MΩ. Cells that show obvious accommodation, an identifying characteristic of pyramidal cells, were used in the study. Labeling the recorded cells exhibiting this characteristic with dye has previously revealed that the recorded cells are indeed pyramidal cells (Sun et al., 1999). Signals were amplified, digitized, and stored using AxoClamp-2B amplifier and DigiData 1200 with the P-clamp data acquisition and analysis software (Axon Instruments, Foster City, CA). Stratum pyramidale, stratum radiatum, and/or stratum oriens were stimulated (about 200 µm from the recording electrode), using bipolar electrodes constructed of Teflon-insulated PtIr wire (25 µm in diameter, the approximate thickness of stratum pyramidale; FHC Inc., Bowdoinham, ME). Monophasic hyperpolarizing postsynaptic potentials (PSPs) were elicited by orthodromic single-pulse stimulation of intemeurons in stratum pyramidale (Collin et al., 1995). In some experiments, a stimulating electrode (about 400 µm from the other stimulating electrodes when two stimulating electrodes were placed) was also placed in stratum oriens to activate cholinergic terminals and evoke acetylcholine release (Cole and Nicoll, 1984), or in stratum radiatum to evoke glutamatergic PSPs. Costimulation of stratum oriens and stratum pyramidale consisted of stimulation of stratum oriens with single pulses (20-60 µA and 50 µs, 1 Hz for 10 s) and stimulation of stratum pyramidale with four trains [10 pulses/train at control intensity (30-60 µA and 50 ms, 100 Hz), starting at the ninth stratum oriens stimulation] at a 0.5-s intertrain interval.

**Drugs and Ligands**. Bicuculline, acetazolamiden (solubilized in dimethyl sulfoxide), kynurenic acid, imidazole, phenylalanine, and atropine outside the scope of the invention were from Sigma (St. Louis, MO) and were solubilized in the noted concentrations and delivered to the slice chamber from an external reservoir. For intralateral ventricular injections of phenylalanine (50 mM), imidazole (0.5 M), and or acetazolamide (10 mM) in vivo, agents (2 µl/site/day) were bilaterally injected during training days about 30 min before the training, at a speed of 1 µl/min. The control rats received the same volume of saline.

**Spatial Maze Tasks.** Effects of increasing HCO₃ formation in vivo on spatial memory were evaluated in rats with the Morris water maze task. Male adult Wistar rats (200-250 g) were housed in a temperature-controlled (20-24°C) room for a week, allowed free access to food and water, and kept on a 12-h light/dark cycle. Rats were anesthetized with sodium pentobarbital (60 mg/kg i.p) and placed in a stereotactic apparatus (Kopf Instruments, Tujunga, CA). The core temperature of rats was monitored and kept constant (38.0 ± 0.5°C) with warming light and pad. Two stainless steel guide cannulas were placed with the tips positioned at the coordinates (anterior-posterior, 0.5 mm; lateral, 1.5 mm; horizontal, 3.2 mm), under aseptic conditions. At the end of surgery and under appropriate anesthesia, rats received (s.c.) banamine (1 mg/kg) and ketoprofen (5 mg/kg) in lactate/Ringer's solution. A 7-day recovery period was allowed before any further experimentation.

On the first day of experiments, all rats were randomly assigned to different groups (10 each) and swam for 2 min in a 1.5- (diameter) X 0.6-m (depth) pool (22 ± 1°C). On the following day, rats were trained in a two-trial per day task for four consecutive days. Each training trial lasted for up to 2 min, during which rats learned to escape from water by finding a hidden platform that was placed at a fixed location and submerged about 1 cm below the water surface. The navigation of the rats was tracked by a videocamera. The escape latency and the route of rats' swimming across the pool to the platform were recorded. The quadrant test (1 min) was performed after removing the platform, 24 h after the last training trial.

Statistical analyses were performed using the Student's *t* test for paired or unpaired data or ANOVA whenever appropriate. The values are expressed as means ± S.E.M., with *n* indicating the number of the cells or rats. All animals used in these experiments were treated under National Institutes of Health guidelines for the welfare of laboratory animals.

### Results

Microstimulation of stratum pyramidale with a single pulse elicited a hyperpolarizing inhibitory postsynaptic potential (IPSP; Fig. 1a). The IPSP was, mainly if not exclusively, from activation of the GABAergic inputs from the Basket intemeurons, whose cell bodies and axons are restricted to stratum pyramidale. As described in previous publications (Sun et al., 1999, 2000), the IPSPs exhibited a reversal potential of about -78 mV. No detectable minor PSP components that exhibit a different reversal potential were observed. Bath application of kynurenic acid (500 µM, 20 min), a broad-spectrum competitive antagonist for both N-methyl-D-aspartate (NMDA) and non-NMDA receptors (Collingridge and Lester, 1989), effectively abolished EPSPs of CA1 pyramidal cells evoked by stimulation of the Schaffer collateral pathways (Sch; by 96.3 ± 4.1%, *n* = 6 from six different rats, *p* < 0:05). At this concentration, kynurenic acid did not increase the IPSP amplitudes (-8.2 ± 0.6 mV prekynurenic acid versus 28.3 ± 0.7 mV during the application; *n* = 7 from seven different rats, *p* > 0.05; Fig. 1a), suggesting that the single-pulse stratum pyramidale micro-stimulation did not evoke a significant glutamatergic EPSP component. The IPSPs, however, were blocked by bicuculline, the selective GABA_{A} receptor antagonist (by 97.9 ± 4.4% on average, *n* = 6 from six different rats, *p* < 0.05; 1 µM, 30-min perfusion; Fig. 1b), indicating that the IPSPs were predominantly mediated by activation of the GABA_{A} receptors and were therefore referred to as Basket interneuron-CA1 responses.

Single-pulse stimulation of stratum oriens (1 Hz, 10 s) coincident with trains of stimulation of stratum pyramidale produced a small but lasting decrease in the IPSP amplitudes (Fig. 1, d and f). For instance, at 40 min after the costimulation, the peak IPSPs were -4.9 ± 0.7 mV, significantly smaller than -7.4 ± 0.9 mV before the associated stimulation *(n* = 8 from seven different rats, *p* < 0.05; paired t test). Two carbonic anhydrase activators, imidazole (100 µM, 20 min; Parkes and Coleman, 1989) or phenylalanine (100 µM, 20 min; Clare and Supuran, 1994), were applied. In the presence of phenylalanine, the peak IPSPs in response to single-pulse stimulation of stratum pyramidale were slightly but significantly reduced (Fig. 1c; to -4.5 ± 0.8 mV in the presence of phenylalanine from prephenylalanine IPSPs of -7.6 ± 1.2 mV; *n* = 7 from seven different rats, *p* < 0.05). In the presence of the carbonic anhydrase activator, the same intensities of costimulation of stratum pyramidale and stratum oriens produced a lasting reversal of the IPSPs to EP-SPs, observed when the membrane potentials were maintained at their control levels (Fig. 1, d and e). Thus, 40 min after the costimulation (under *Materials and Methods*) and in the presence of phenylalanine, the peak PSPs were 6.4 ± 1.1 mV, significantly different *(n* = 8 from eight different rats, *p* < 0.05) from their prephenylalanine values (-7.2 ± 1.2 mV) or from those in the presence of phenylalanine but before the costimulation (Fig. 1d). In the presence of imidazole, similar effects on the IPSPs (-5.3 ± 0.7 mV in the presence of imidazole versus preimidazole of -7.8 ± 0.6 mV; *n* = 7 from seven different rats, *p* < 0.05) and effects of the costimulation (peak PSPs: 4.2 ± 0.6 mV, in the presence of imidazole and 40 min after the costimulation versus preimidazole values of -7.5 ± 0.7 mV; *n* = 6 from six different rats, *p* < 0.05) were observed, although in general, less potent. Thus, the results with imidazole were not illustrated in detail.

Both the reducing effect of carbonic anhydrase activators on the IPSPs and the synaptic switching effect with costimulation of the cholinergic and GABAergic inputs depend on activity of the carbonic anhydrase. For instance, in the presence of acetazolamide (10 µM, 20 min), a blocker of carbonic anhydrase and thus the synthesis of HCO₃ (Staley et al., 1995), phenylalanine did not significantly reduce the peak IPSPs (-7.7 ± 0.9 mV in the presence of phenylalanine versus prephenylalanine peak IPSPs of -7.9 ± 1.1 mV, *n* = 6 from six different rats, *p* > 0.05). Nor did imidazole, in the presence of acetazolamide, significantly change the size of the IPSPs (-7.5 ± 1.0 mV in the presence of imidazole versus preimidazole peak IPSPs of -7.4 ± 0.8 mV, *n* = 5 from five different rats, *p* > 0.05). The same intensities of costimulation did not induce the synaptic switch (Fig. 1, d and g) in the presence of acetazolamide and phenylalanine or imidazole. Thus, in the presence of acetazolamide and phenylalanine, these IPSPs were not significantly altered by the costratum oriens stratum pyramidale stimulation (-7.8 ± 1.3 mV, 40 min after compared with -7.6 ± 0.9 mV control value, *n* = 8 from eight different rats, *p* > 0.05). Furthermore, the co-stimulation did not significantly alter the IPSPs in the presence of acetazolamide and imidazole (-7.7 ± 1.1 mV, 40 min after compared with -7.5 ± 0.8 mV control value, *n* = 6 from six different rats, *p* > 0.05).

The influence of the GABAergic synaptic switch on the signal passage through the CA1 cells was evaluated when the glutamatergic Sch inputs were costimulated. In eight cells, single-pulse stratum pyramidale stimulation evoked an IPSP (Fig. 2a). Excitatory Sch input was stimulated at intensities 30% above the action potential threshold (100% of 20 trials) of the recorded cells (Fig. 2b). Costimulation of the GABAergic inputs and Sch blocked (100% of 20 trials; *n* = 10 from eight different rats, *p* > 0.05) the effects of excitatory Sch input, stimulated at above-action-potential-threshold intensities (Fig. 2c) in all eight cells tested. The effective signal-filtering period in each single-pulse-evoked inhibitory response was ≥ 100 ms, during which no action potential (0% of 20 trials) was evoked by Sch stimulation at the above-thresh-old intensity. After the synaptic switch (Fig. 2d) induced by costimulation of the GABAergic and cholinergic inputs in the presence of phenylalanine, below-threshold Sch stimulation, which by itself did not evoke action potentials (0% of 20 trials; Fig. 2e), became sufficient to evoke action potentials (100% of 20 trials; *n*= 8 from eight different rats, *p* < 0.05) when delivered during the period of $100 ms of single-pulse stimulation of the GABAergic input (Fig. 2f; *n* = 8 from eight different rats). Multiple spikes were evoked when the Basket interneurons-CA1 PSP was costimulated with above-thresh-old Sch stimulation after inducing the synaptic switch (data not shown). Thus, after the synaptic switch, activity of the GABAergic intemeurons amplified excitatory Sch inputs. Therefore, weak signals are amplified after synaptic switch o trigger action potentials, while strong excitatory signals cannot successfully pass through the network under associated inhibition.

The effects of carbonic anhydrase activators were tested on spatial learning in rats, using the hidden-platform water maze. As shown in Fig. 3a, the latency to escape to the platform in all three groups of rats decreased following the training sessions. Statistical analysis revealed significant effects of groups (F_{2,27} = 9.192, *p* < 0.001), trials (F_{7,218} = 7.83, *p* < 0.001), and groups X session of trials (F_{14,218} = 3.70, *p* < 0.001), indicating that spatial learning in rats injected with phenylalanine (phenylalanine rats) was faster than in rats injected with saline (control rats). Moreover, a post hoc analysis reveals a significant difference from the second to sixth trials *(p* < 0.05), confirming better learning in phenylalanine rats. In fact, the escape latency of the phenylalanine rats reached a plateau on the fifth trial. Three additional trials were needed for the control rats to show the same escape latency as the phenylalanine rats (Fig. 3a). Quadrant tests 24 h after the last training trial revealed that the control rats *(*F_{3,36} = 159.9, *p* < 0.0001; ANOVA and Newman-Keuls post hoc test), and the phenylalanine rats (F_{3,36} = 201.2, *p* < 0.0001) spent more time searching in the target quadrant (quadrant 4) where the platform was previously placed and had been removed. However, in comparison with control rats, phenylalanine rats exhibited a clearly greater preference for the target quadrant (by 24.8 ± 1.8%, *p* < 0.05; unpaired *t* test) (Fig. 3, d and e). The target quadrant ratios, target/average of the nontarget quadrants, between the pheynlalanine and the control rats were significantly different *(p* < 0.001; Fig. 3b). Similarly, rats injected with imidazole (imidazole rats) also showed a faster learning and a significant shorter escape latency from the third to sixth trials *(p* < 0.05) than the control animals. Quadrant tests revealed that imidazole rats had a greater preference for the target quadrant (by 15.1 ± 1.6%, *p* < 0.05) than the control rats. Thus, the rats injected with the carbonic anhydrase activators performed better than their controls in this spatial memory retention task. The average swim speeds for all eight trials, however, did not differ between all the groups (Fig. 3c; *p* > 0.05), including the imidazole and acetazol-amide/imidazole groups (data not shown), indicating that the carbonic anhydrase activators and inhibitor did not grossly affect their sensory or locomotor activities. During the experimental periods, no rats showed any apparent sign of discomfort or abnormal behaviors such as hypo- or hyperactivity.

The effects of carbonic anhydrase activators on spatial learning were sensitive to carbonic anhydrase inhibitors. Bilateral intraventricular injections of acetazolamide not only eliminated the effects of the carbonic anhydrase activators on the learning but also produced memory impairment (Fig. 3a). The acetazolamide/phenylalanine group showed a strikingly smaller reduction (F_{1,18} = 40.38, *p* < 0.0001) in escape latency during training trials than the control group did. Quadrant tests revealed that the acetazolamide/phenylalanine rats showed no significantly different preference for a particular quadrant (F_{3,36} = 1.43, *p* > 0.05; Fig. 3f) and a significantly different *(p* < 0.001) target quadrant ratio from those of the phenylalanine and the control rats (Fig. 3b). Identical results were also observed in rats injected with acetazolamide and imidazole (data not shown).

According to the invention, enhancement of the GABAergic synaptic switch in controlling signal processing in the hippocampal network can be achieved through the use of carbonic anhydrase activators, and these carbonic anhydrase activators increase efficacy of temporally associated activity of the cholinergic and GABAergic inputs in switching the hyperpolarizing GABAergic IPSPs to excitatory PSPs. The synaptic switch can be induced by associative postsynaptic stimulation (Collin et al., 1995), activation of the calexcitin signal cascade, or costimulation of the cholinergic and GABAergic inputs at greater intensities and more prolonged periods of stimulation (Sun et al., 2001a). The results shown above indicate that the presence of the enzyme activators facilitates induction of the synaptic switch so that weaker and fewer trains of costimulation were required. Thus, administration of carbonic anhydrase activators may additively or synergistically augment a naturally occurring activation of carbonic anhydrase that occurs in neurons of pathways associated with attentive cognition.

Two enzyme activators from different classes of compounds, which have different spectra of biological actions, were used in the study, yielding similar results. They were administered directly into the brain to avoid the limitation of accumulation in the brain by the blood-brain barrier. Competitive transport and rapid peripheral hydroxylation are known to limit the phenylalanine concentration in the brain of systemically administered phenylalanine-containing substances (such as aspartame, whose metabolites include 5-benzyl-3,6-dioxo-2-piperazineacetic acid, phenylalanyl aspartic acid, asparaginyl-phenylalanine, phenylalanine methyl ester, phenylalanine, aspartic acid, methanol, and formate). These effects limit phenylalanine's access to the brain and possibly its behavioral impact. In addition to activation of carbonic anhydrase, high concentration of phenylalanine in the brain might facilitate the synthesis of catecholamines and catecholaminergic transmission.

Imidazole-like structures, on the other hand, may react with many biologically active molecules, including monoamine oxidase, histamine H₂ receptors, angiotensin II type 1 receptors, ethanol binding sites in GABA receptor channel complex, GABA_{c} receptors, the nicotinic-cholinergic receptor channel complex, the prosthetic heme group of the nitric-oxide synthase, some K_{ATP} channels, and imidazole binding sites. The biological consequences and specificity of an increased brain imidazole concentration, therefore, still remain to be clarified. Thus, these results do not rule out a possible contribution of synaptic/signal interaction in other brain regions or an action of the substances and their metabolites at the α-adrenoceptors, dopaminergic receptors, and/or histaminergic receptors to the enhancement of spatial learning and memory. The common denominator of the two carbonic anhydrase activators, the action on carbonic anhydrase, however, is the likely underlying mechanism for the observed effects. The critical role of carbonic anhydrase activation in the observed effects of carbonic anhydrase activators was further directly demonstrated by the effectiveness of acetazolamide, a carbonic anhydrase inhibitor, in blocking the synaptic switch. Acetazolamide has been shown to be able to reduce or eliminate flux of HCO₃ in hippocampal pyramidal neurons underlying a depolarizing PSP (Staley et al., 1995). Activity of carbonic anhydrase in the CA1 pyramidal cells is essential since intracellular application of benzolamide, a membrane-impermeant carbonic anhydrase inhibitor, was previously found to effectively block the GABAergic synaptic switch (Sun et al., 1999).

Carbonic anhydrase is a highly efficient enzyme. If its activity is crucial for coding and storing learned information, one would expect the existence of cellular mechanisms to control activity of the enzyme. There are indications that intracellular Ca²⁺ release increases HCO₃ conduction through the GABA_{A} receptor-mediated IPSPs and that the effect is sensitive to carbonic anhydrase inhibition (Sun et al., 2000). Membrane association is another efficient mechanism to activate carbonic anhydrase (Parkes and Coleman, 1989). Translocation and membrane association of the cytosol carbonic anhydrase may participate in memory acquisition and/or consolidation. The inventive method permits activation of neuronal carbonic anhydrase by any or all such mechanisms. The involvement of carbonic anhydrase in cognitive functions is consistent with the evidence (Meier-Ruge et al., 1984) of a significantly diminished activity of the enzyme in Alzheimer's disease than in age-matched controls and with increasing age.

The present results demonstrate that the switched synaptic responses provide a postsynaptic mechanism to direct or gate signal flow through the hippocampal network. The GABAergic intemeurons, especially the Basket intemeurons, whose cell bodies and axons are restricted in the cell layer, are known to innervate the perisomatic region of the pyramidal cells. Thus, bursting activity from the intemeurons in the absence of synaptic switch inhibits the pyramidal cells, powerfully blocking excitatory signal transfer through the hippocampal circuit. An associated activation of the cholinergic and GABAergic inputs can trigger the synaptic switch, especially when the carbonic anhydrase is activated. After the synaptic switch, however, the same type of GABAergic activity amplifies excitatory signal. The mechanism thus differentiates responses according to the nature and temporal association of relevant signals and the neural activity states, a phenomena that may underlie synaptic plasticity in learning and memory (Liu and Cull-Candy, 2000; Shulz et al., 2000). The synaptic switch mechanism enables the network to perform signal processing and gate information flow and direction accordingly.

Thus according to the invention, altering the neural activity states that learning depends on via carbonic anhydrase activity represents an effective therapeutic strategy to achieve memory therapy. Agents that activate carbonic anhydrase according to the invention have clinical value for enhanced memory and for the treatment of spatial memory decline. Phenylalanine (reference) may be used in the majority of individuals who do not have genetic lack of phenylalanine hydroxylase, and more potent and selective nonphenylalanine activators (such as imidazole-and histamine-derivatives) can help individuals with hydroxylase dysfunction.

### REFERENCES

1. Alkon DL, Nelson TJ, Zhao WQ and Cavallaro S (1998) Time domains of neuronal Ca2+ signaling and associative memory: steps through a calexcitin, ryanodine receptor, K+ channel cascade. Trends Neurosci 21:529-537.
2. Alkon DL, Sanchez-Andres JV, Ito E, Oka K, Yoshioka T and Collin C (1992) Long-term transformation of an inhibitory into an excitatory GABAergic synaptic response. Proc Natl Acad Sci USA 85:11862-11866.
3. Banks M, White JA and Pearce RA (2000) Interactions between distinct GABAA circuits in hippocampus. Neuron 25:449-57.
4. Bartus RT, Reginald L, Dean RL, Beer B and Lippa AS (1982) The cholinergic hypothesis of geriatric memory dysfunction. Science (Wash DC) 217:408-414.
5. Buhl EH, Cobb SR, Halasy K and Somogyi P (1995) Properties of unitary IPSPs evoked by anatomically identified basket cells in the rat hippocampus. Eur J Neurosci 7:1989-2004.
6. Burg M, Heinemann U and Schmitz D (1998) Neuroactive steroids induce GABAA receptor-mediated depolarizing postsynaptic potentials in hippocampal CA1 pyra-midal cells of rats. Eur J Neurosci 10:2880-2896.
7. Christie BR, Kerr DS and Abraham WC (1994) Evidence for common expression mechanisms underlying heterosynaptic and associative long-term depression in the dentate gyrus. Hippocampus 4:127-135.
8. Clare BW and Supuran CT (1994) Carbonic anhydrase activators. 3: Structure-activity correlations for a series of isozyme II activators. J Pharmacol Sci 83:768-773.
9. Cobb SR, Buhl EH, Halasy K, Paulsen O and Somogyi P (1995) Synchronization of neuronal activity in hippocampus by individual GABAergic intemeurons. Nature (Lond) 378:75-78.
10. Cole AE and Nicoll RA (1984) Characterization of a slow cholinergic post-synaptic potential recorded in vitro from rat hippocampal pyramidal cells. J Physiol (Lond) 352:173-188.
11. Collin C, Devane WA, Dahl D, Lee C-J, Axelrod J and Alkon DL (1995) Long-term synaptic transmission of hippocampal CA1 γ-aminobutyric acid synapses and the effect of anandamide. Proc Natl Acad Sci USA 92:10167-10171.
12. Collingridge GL and Lester RA (1989) Excitatory amino acid receptors in the vertebrate central nervous system. Pharmacol Rev 40:143-209.
13. Cooper JD and Sofroniew MV (1996) Increased vulnerability of septal cholinergic neurons to partial loss of target neurons in aged rats. Neuroscience 75:29-35.
14. Dickinson-Anson H, Aubert I, Gage FH and Fisher LJ (1998) Hippocampal grafts of acetylcholine-producing cells are sufficient to improve behavioral performance following a unilateral fimbria-fornix lesion. Neuroscience 84:771-781.
15. Kaila K, Lamsa K, Smirnov S, Taira T and Voipio J (1997) Long-lasting GABA-mediated depolarization evoked by high-frequency stimulation in pyramidal neurons of rat hippocampal slice is attributable to a network-driven, bicarbonate-dependent K+ transient. J Neurosci 17:7662-7672.
16. Kornhauser JM and Greenberg ME (1997) A kinase to remember: dual roles for MAP kinase in long-term memory. Neuron 18:839-842.
17. Leinekugel X, Khalilov I, McLean H, Caillard O, Gaiarsa JL, Ben-Ari Y and Khazi-pov R (1999) GABA is the principal fast-acting excitatory transmitter in the neonatal brain. Adv Neurol 79:189-201.
18. Lindskog, "Structure and Function of Carbonic Anhydrase, "Pharmacol. Ther. Vol.74 (1), P1-20, 1997.
19. Liu S-QJ and Cull-Candy SG (2000) Synaptic activity at calcium-permeable AMPA receptors induces a switch in receptor subtype. Nature (Lond) 405:454-458.
20. Meier-Ruge W, Iwangoff P and Reichlmeier K (1984) Neurochemical enzyme changes in Alzheimer's and Pick's disease. Arch Gerontol Geriatr 3:161-165.
21. Ohno M, Kobayashi M, Kishi A and Watanabe S (1997) Working memory failure by combined blockade of muscarinic and β-adrenergic transmission in the rat hip-pocampus. Neuroreport 8:1571-1575.
22. Parkes JL and Coleman PS (1989) Enhancement of carbonic anhydrase activity by erythrocyte membranes. Arch Biochem Biophys 275:459-468.
23. Pasternack M, Voipio J and Kaila K (1993) Intracellular carbonic anhydrase activity and its role in GABA-induced acidosis in isolated rat hippocampal pyramidal neurons. Acta Physiol Scand 148:229-231.
24. Paulsen O and Moser EI (1998) A model of hippocampal memory encoding and retrieval: GABAergic control of synaptic plasticity. Trends Neurosci 21:273-278.
25. Perry E, Walker M, Grace J and Perry R (1999) Acetylcholine in mind: a neurotransmitter correlate of consciousness? Trends Neurosci 22:273-280.
26. Shulz DE, Sosnik R, Ego V, Haidarliu S and Ahissar E (2000) A neuronal analogue of state-dependent learning. Nature (Lond) 403:549-553.
27. Sone M, Sei H, Morita Y, Ogura T and Sone S (1998) The effects of acetazolamide on arterial pressure variability during REM sleep in the rat. Physiol Behav 63:213-218.
28. Staley KJ, Soldo BL and Proctor WR (1995) Ionic mechanisms of neuronal excitation by inhibitory GABAA receptors. Science (Wash DC) 269:977-981.
29. Steckler T, Sahgal A, Aggleton JP and Drinkenburg WH (1998) Recognition memory in rats. III. Neurochemcial substrates. Prog Neurobiol 54:333-348.
30. Sun MK, Alkon DL., "Pharmacological Enhancement of Synaptic Efficacy, Spatial Learning, and Memory Through Carbonic anhydrase Activation in Rats," JPET 297:961-967 (2001).
31. Sun M-K, Zhao W-Q, Neslon TJ and Alkon DL (2001b) Theta rhythm of CA1 hippocampal neuron activity: gated by GABAergic synaptic depolarization. J Neu-rophysiology 85:269-279.
32. Sun, M.K., Dennis Dahl and Daniel L. Alkon, "Heterosynaptic Transformation of GABAergic Gating in the Hippocampus and Effects of Carbonic Anhydrase Inhibition," JPET 2001;297(3): 811-817, March 2001
33. Sun, M.K., Thomas J. Nelson, and Daniel L. Alkon, "Functional switching of GABAergic synapses by ryanodine receptor activation," Proc. Natl. Acad. Sci. USA vol. 97, pp. 12300-12305 (October 24, 2000).
34. Sun, M.K., Thomas J. Nelson, Hui Xu, and Daniel L. Alkon, "Calexcitin transformation of GABAergic synapses: From excitation filter to amplifier," Proc. Natl. Acad. Sci. USA vol. 96, pp. 7023-7028 (June 8, 1999).
35. Taira T, Lamsa K and Kaila K (1997) Posttetanic excitation mediated by GABAA receptors in rat CA1 pyramidal neurons. J Neurophysiol 77:2213-2218.
36. Tang YP, Shimizu E, Dube GR, Rampon C, Kerchner GA, Zhuo M, Liu G and Tsien JZ (1999) Genetic enhancement of learning and memory in mice. Nature (Lond) 401:63-69.
37. Winkler J, Suhr ST, Gage FH, Thal LJ and Fisher LJ (1995) Essential role of neocortical acetylcholine in spatial memory. Nature (Lond) 375:484-487.
38. Winson J (1978) Loss of hippocampal theta rhythm results in spatial memory deficit in the rat. Science (Wash DC) 201:160-163.
39. Wong RK and Watkins DJ (1982) Cellular factors influencing GABA response in hippocampal pyramidal cells. J Neurophysiol 48:938-951.
40. Wu M, Shanabrough M, Leranth C and Alreja M (2000) Cholinergic excitation of septohippocampal GABA but not cholinergic neurons: implications for learning and memory. J Neurosci 20:3900-3908.
41. Xiang Z, Huguenard JR and Prince DA (1998) Cholinergic switching within neocortical inhibitory networks. Science (Wash DC) 281:985-988.

## Claims

1. A carbonic anhydrase activator compound for improving attentive cognition in a dose effective to improve attentive cognition when administered to the brain of a subject in need of improved attentive cognition, the carbonic anhydrase activator compound being defined by structure I: 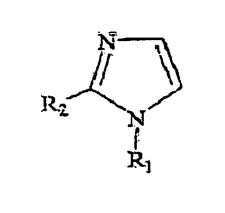 wherein R¹ and R² are independently H or linear, branched or cyclic C₁-C₆ alkyl or C₁-C₄ alkyl; or
wherein R¹ is 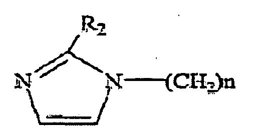 and *n* is 1 or 2 and R² is H or linear, branched or cyclic C₁-C₆ alkyl or C₁-C₄ alkyl; and salts thereof.

2. The carbonic anhydrase activator compound of claim 1, wherein the compound potentiates intraneuronal carbonic anhydrase activity.

3. The carbonic anhydrase activator compound of claim 1, wherein the activator has structure I and wherein R¹ is H, methyl or ethyl; and R² is H or methyl.

4. The carbonic anhydrase activator compound of claim 1, wherein *n* is 1 or 2; and R² is H or methyl.

5. The carbonic anhydrase activator compound of claim 1, wherein the activator is selected from the group consisting of imidazole, linked di-imidazole, and salts thereof.

6. The carbonic anhydrase activator compound of claim 1, wherein the carbonic anhydrase activator is to be administered as a pharmaceutical composition or in a pharmaceutically acceptable carrier.

7. The carbonic anhydrase activator compound of claim 1, wherein the patient has a neurodegenerative disorder.

8. The carbonic anhydrase activator compound of claim 1, wherein the compound enhances cognitive ability, attention, learning, and/or memory in individuals without a neurological disorder.

9. The carbonic anhydrase activator compound of claim 1, wherein the compound facilitates establishement of a theta rhythm via bicarbonate-mediated GABAergic depolarization.

10. The carbonic anhydrase activator compound of claim 9, wherein the compound improves memory formation, learning, spatial memory, and/or attention.

11. The carbonic anhydrase activator compound of claim 9, wherein the compound intervenes in the intracellular signaling cascade responsible for the theta rhythm, the intervention comprising modulating HCO₃ conductance by directly altering intraneuronal carbonic anhydrase activity.

12. The carbonic anhydrase activator compound of claim 11, wherein the intervention modulates the HCO₃⁻ current relative to the Cl⁻ and K⁺ currents.

13. The carbonic anhydrase activator compound of claim 1, wherein the compound improves attentive cognition in a subject with Alzheimer's disease, stroke, hypoxia, and/or ischemia.

14. The carbonic anhydrase activator compound of claim 1, wherein the compound is one that provides carbonic anhydrase activity at least 150% that of alanine in vitro.

15. The carbonic anhydrase activator compound of claim 1, wherein the compound is one that provides carbonic anhydrase activity at least 200% that of alanine in vitro.

16. The carbonic anhydrase activator compound of claim 1, wherein the compound is one that provides carbonic anhydrase activity at least 250% that of alanine in vitro.

## Patentansprüche

1. Carboanhydraseaktivatorverbindung zur Verbesserung der Aufmerksamkeitswahrnehmung mit einer Dosis, die wirksam ist, die Aufmerksamkeitswahrnehmung zu verbessern, wenn sie an das Hirn von einem Subjekt verabreicht wird, das einer verbesserten Aufmerksamkeitswahrnehmung bedarf, wobei die Carboanhydraseaktivatorverbindung durch die Struktur I definiert ist: 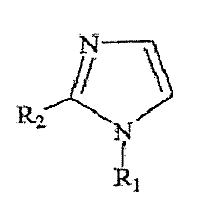 worin R¹ und R² unabhängig H oder ein lineares, verzweigtes oder zyklisches C₁-C₆-Alkyl oder C₁-C₄-Alkyl sind; oder
worin R¹ 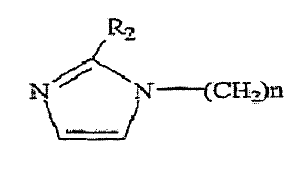 ist
und *n* 1 oder 2 ist und R² H oder ein lineares, verzweigtes oder zyklisches C₁-C₆-Alkyl oder C₁C₄-Alkyl ist; und die Salze davon.

2. Carboanhydraseaktivatorverbindung nach Anspruch 1, worin die Verbindung die intraneuronale Carboanhydraseaktivität verstärkt.

3. Carboanhydraseaktivatorverbindung nach Anspruch 1, worin der Aktivator die Struktur I hat und worin R¹ H, Methyl oder Ethyl ist; und R² H oder Methyl ist.

4. Carboanhydraseaktivatorverbindung nach Anspruch 1, worin *n* 1 oder 2 ist; und R² H oder Methyl ist.

5. Carboanhydraseaktivatorverbindung nach Anspruch 1, worin der Aktivator aus der Gruppe ausgewählt ist, bestehend aus Imidazol, verbundenes Di-Imidazol und die Salze davon.

6. Carboanhydraseaktivatorverbindung nach Anspruch 1, worin der Carboanhydraseaktivator als eine pharmazeutische Zusammensetzung oder in einem pharmazeutischen Träger verabreicht wird.

7. Carboanhydraseaktivatorverbindung nach Anspruch 1, worin der Patient eine neurodegenerative Krankheit hat.

8. Carboanhydraseaktivatorverbindung nach Anspruch 1, worin die Verbindung die kognitive Fähigkeit, die Aufmerksamkeit, das Lernen und/oder das Gedächtnis in Individuen ohne eine neurologische Krankheit steigert.

9. Carboanhydraseaktivatorverbindung nach Anspruch 1, worin die Verbindung die Etablierung eines Theta-Rhythmus über eine bicarbonatvermittelte GABAgene Depolarisation unterstützt.

10. Carboanhydraseaktivatorverbindung nach Anspruch 9, worin die Verbindung die Gedächtnisbildung, das Lernen, das räumliche Gedächtnis und/oder die Aufmerksamkeit verbessert.

11. Carboanhydraseaktivatorverbindung nach Anspruch 9, worin die Verbindung in der intrazellulären Signalkaskade eingreift, die für den Theta-Rhythmus verantwortlich ist, wobei das Eingreifen die Modulation der HCO₃⁻-Leitfähigkeit umfasst, indem direkt die intraneuronale Carboanhydraseaktivität verändert wird.

12. Carboanhydraseaktivatorverbindung nach Anspruch 11, worin das Eingreifen den HCO₃⁻-Strom relativ zu den Cl⁻- und K⁺-Strömen moduliert.

13. Carboanhydraseaktivatorverbindung nach Anspruch 1, worin die Verbindung die Aufmerksamkeitswahrnehmung in einem Subjekt mit der Alzheimerschen Krankheit, einem Schlaganfall, einer Hypoxie und/oder Ischämie verbessert.

14. Carboanhydraseaktivatorverbindung nach Anspruch 1, worin die Verbindung eine ist, die eine Carboanhydraseaktivität von mindestens 150 % von der von Alanin in vitro liefert.

15. Carboanhydraseaktivatorverbindung nach Anspruch 1, worin die Verbindung eine ist, die eine Carboanhydraseaktivität von mindestens 200 % von der von Alanin in vitro liefert.

16. Carboanhydraseaktivatorverbindung nach Anspruch 1, worin die Verbindung eine ist, die eine Carboanhydraseaktivität von mindestens 250 % von der von Alanin in vitro liefert.

## Revendications

1. Composé activateur d'anhydrase carbonique pour améliorer la cognition attentive à une dose efficace pour améliorer la cognition attentive lorsqu'il est administré au cerveau d'un sujet nécessitant une cognition attentive améliorée, le composé activateur d'anhydrase carbonique étant défini par la structure I : 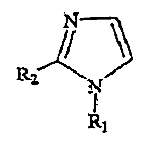 dans laquelle R¹ et R² représentent indépendamment H ou un groupe alkyle en C₁₋₆ ou alkyle en C₁₋₄ linéaire, ramifié ou cyclique ; ou
dans laquelle R¹ représente 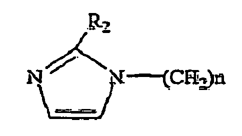 et *n* vaut 1 ou 2 et R² représente H ou un groupe alkyle en C₁₋₆ ou alkyle en C₁₋₄ linéaire, ramifié ou cyclique ; et leurs sels.

2. Composé activateur d'anhydrase carbonique selon la revendication 1, dans lequel le composé potentialise l'activité d'anhydrase carbonique intraneuronale.

3. Composé activateur d'anhydrase carbonique selon la revendication 1, dans lequel l'activateur a la structure I et dans lequel R¹ représente H, un groupe méthyle ou éthyle ; et R² représente H ou un groupe méthyle.

4. Composé activateur d'anhydrase carbonique selon la revendication 1, dans lequel *n* vaut 1 ou 2 ; et R² représente H ou un groupe méthyle.

5. Composé activateur d'anhydrase carbonique selon la revendication 1, dans lequel l'activateur est choisi dans le groupe consistant en imidazole, di-imidazole lié, et leurs sels.

6. Composé activateur d'anhydrase carbonique selon la revendication 1, dans lequel l'activateur d'anhydrase carbonique est à administrer sous forme d'une composition pharmaceutique ou dans un véhicule pharmaceutiquement acceptable.

7. Composé activateur d'anhydrase carbonique selon la revendication 1, le patient présentant une affection neurodégénérative.

8. Composé activateur d'anhydrase carbonique selon la revendication 1, le composé augmentant la capacité cognitive, l'attention, l'apprentissage et/ou la mémoire chez des individus sans affection neurologique.

9. Composé activateur d'anhydrase carbonique selon la revendication 1, le composé facilitant l'établissement d'un rythme theta par dépolarisation GABAergique médiée par le bicarbonate.

10. Composé activateur d'anhydrase carbonique selon la revendication 9, le composé améliorant la formation de la mémoire, l'apprentissage, la mémoire spatiale et/ou l'attention.

11. Composé activateur d'anhydrase carbonique selon la revendication 9, le composé intervenant dans la cascade de signalisation intracellulaire responsable du rythme theta, l'intervention comprenant la modulation de la conductance de HCO₃⁻ en altérant directement l'activité anhydrase carbonique intraneuronale.

12. Composé activateur d'anhydrase carbonique selon la revendication 11, l'intervention modulant le courant de HCO₃⁻ par rapport aux courants de Cl⁻ et de K⁺.

13. Composé activateur d'anhydrase carbonique selon la revendication 1, le composé améliorant la cognition attentive chez un sujet ayant la maladie d'Alzheimer, un accident cérébrovasculaire, une hypoxie et/ou une ischémie.

14. Composé activateur d'anhydrase carbonique selon la revendication 1, le composé étant un composé qui fournit une activité anhydrase carbonique égale à au moins 150 % de celle de l'alanine in vitro.

15. Composé activateur d'anhydrase carbonique selon la revendication 1, le composé étant un composé qui fournit une activité anhydrase carbonique égale à au moins 200 % de celle de l'alanine in vitro.

16. Composé activateur d'anhydrase carbonique selon la revendication 1, le composé étant un composé qui fournit une activité anhydrase carbonique égale à au moins 250 % de celle de l'alanine in vitro.
